# EUROPEAN PATENT SPECIFICATION

(11) **EP 1 145 712 B1**
(45) Date of publication and mention of the grant of the patent: **02.11.2005**
(21) Application number: 01112778.4
(22) Date of filing: 21.03.1995
(51) Int. Cl.: A61K 9/20

(54) **Sustained release excipient**
Trägerstoff zur verzögerten Freisetzung
Excipient à libération prolongée

(30) Priority: 25.04.1994 US 232625
(43) Date of publication of application: 17.10.2001
(62) Divisional of application: 95914917.0
(73) Proprietor: Penwest Pharmaceuticals Co., Patterson, NY 12563-9970 (US)
(72) Inventor: Baichwal, Anand R., Wappinger Falls, NY 12590 (US); McCall, Troy W., Germantown, TN 38138 (US)
(74) Representative: Gill, Siân Victoria

(56) References cited:
- EP-A- 0 265 116
- EP-A- 0 311 582
- EP-A- 0 341 745
- EP-A- 0 360 562
- EP-A- 0 642 785
- WO-A-95/13055
- WO-A-95/28917
- GB-A- 2 264 866
- US-A- 4 309 405
- US-A- 4 764 380
- US-A- 5 128 143

## Description

### FIELD OF THE INVENTION

The present invention relates to sustained release excipient formulations which may be blended with a wide range of therapeutically active medicaments and made into sustained release oral solid dosage forms.

### BACKGROUND OF THE INVENTION

In our U.S. Patent Nos. 4,994,276; 5,128,143; and 5,135,757, we reported that a controlled release excipient which is comprised of synergistic heterodisperse polysaccharides (e.g., a heteropolysaccharide such as xanthan gum in combination with a polysaccharide gum capable of cross-linking with the heteropolysaccharide, such as locust bean gum) is capable of processing into oral solid dosage forms using either direct compression, following addition of drug and lubricant powder, conventional wet granulation, or a combination of the two. The release of the medicament from the formulations therein proceeded according to zero-order or first-order mechanisms.

The controlled release excipients disclosed in U.S. Patent Nos. 4,994,276, 5,128,143, and 5,135,757 are commercially available under the tradename TIMERx™ from Edward Mendell Co., Inc., Patterson, N.Y., which is the assignee of the present invention.

European Pat. No. 234670 B (Pankhania et al.) describes a sustained-release pharmaceutical formulation containing xanthan gum wherein the xanthan gum comprises from about 7.5 to about 28 percent, by weight, of the formulation except for a formulation wherein the sustained release carrier comprises a mixture of 15-50 parts by weight dimethylsiloxane, 30-100 parts by weight silicic acid, 30-100 parts by weight mannans or galactans or a mixture thereof, 50-150 parts by weight xanthans and 5-75 parts by weight micronized seaweed.

European Publication No. 0 642 785A, which falls under the provisions of Article 54(3) EPC, discloses sustained release heterodisperse hydrogel systems for insoluble drugs, including a gelling agent comprising a combination of a heteropolysaccharide gum and a homopolysaccharide gum, and a cationic cross-linking agent.

### OBJECTS AND SUMMARY OF THE INVENTION

It is an object of the present invention to provide new sustained release matrices which, when incorporated into a final product, may cause the release of therapeutically active medicaments over an extended period of time when the dosage form is exposed to fluids in an environment of use, e.g. from about 12 to about 24 hours or more.

In accordance with the above-mentioned object, and others which will be apparent from the following disclosure, the present invention relates to an oral dosage form comprising a therapeutically active agent and a sustained release excipient, the excipient comprising: a gum component consisting of xanthan gum, the percentage of said gum being from 10 to 40 percent of the sustained release excipient; a cationic cross-linking agent capable of cross-linking said gum in the presence of aqueous solutions, the ratio of said gum to said cationic cross-linking agent being from 1:1 to 3.5:1; an inert pharmaceutical diluent selected from the group consisting of a monosaccharide, a disaccharide, a polyhydric alcohol, a cellulose, a starch, and mixtures thereof, the percentage of said inert diluent being from 60 to 85 percent by weight, of the sustained release excipient.

In certain preferred embodiments, the cationic cross-linking agent comprises from 1 to 20 percent by weight of the sustained release excipient.

The sustained-release matrices of the present invention can be mixed with a wide range of therapeutically active medicaments and thereafter compressed into solid dosage forms such as tablets. The solid dosage forms thus made slowly release the medicament over about a 24-hour time period when ingested and exposed to an environment of use, e.g. gastric fluids. By varying the amount of excipient relative to the medicament, a desired sustained-release profile can be attained.

In a preferred embodiment the cationic cross-linking agent is calcium sulfate.

The present invention also provides a 24-hour sustained-release tablet for oral administration comprising (I) a hydrophilic material comprising (a) a heteropolysaccharide; or (b) a heteropolysaccharide and a cationic cross-linking agent capable of cross-linking said heteropolysaccharide; and (II) an inert pharmaceutical filler comprising from 60 to 85 percent by weight of the hydrophilic material; and (III) an effective amount of a therapeutically active ingredient.

In addition, the present invention provides a method for providing a sustained release matrix for sustained release dosage forms containing one or more therapeutically active medicaments, comprising preparing a sustained-release matrix by dry blending the requisite amounts of heteropolysaccharide gum, inert pharmaceutical filler, and cationic cross-linking agent. In certain preferred embodiments the sustained release excipient is prepared by dry blending the requisite amounts of heteropolysaccharide gum, inert pharmaceutical filler, and cationic cross-linking agent, wet granulating the mixture, and then drying the mixture to obtain the final sustained release excipient. The sustained release excipient thereby obtained may then be directly admixed with an active ingredient along with any further pharmaceutically necessary inert excipients, and then formulated into a final oral solid dosage form.

After admixture with the therapeutically active medicaments, the sustained release excipient/drug mixture may then be manufactured into a final dosage form, e.g., by directly compressing the mixture into tablets.

### DETAILED DESCRIPTION OF THE INVENTION

The excipients of the present invention have been pre-optimized by providing a sustained-release excipient product which may be mixed with a wide range of medicaments and made into oral solid dosage forms capable of releasing the active medicament in the environment of use over about a 12 to 24 hour time period, without the aid of the usual pharmaceutical dry or wet binders, fillers, disintegrants, glidants etc., which must be added in many prior art compositions to obtain an acceptable solid dosage form. Thus, the excipients of the present invention substantially overcome the need for conducting further experimentation needed to optimize release characteristics and tabletting properties for a particular therapeutically active medicament.

In other words, the controlled release excipient used in the present invention provides a product which contains a combination of ingredients in preselected proportions to each other which provides a desired controlled release profile over a 12 to at least a 24-hour period for a wide variety of drugs. Thus, once the excipient product is admixed with an active medicament (and preferably with a lubricant) in a ratio to the sustained-release excipient in accordance with the present invention, the resulting mixture may be made into oral solid dosage forms capable of releasing an active medicament over an extended period of time.

Xanthan gum is produced by microorganisms, for instance, by fermentation with the organism xanthomonas compestris. Most preferred is xanthan gum which is a high molecular weight (>10⁶) heteropolysaccharide. Xanthan gum contains D-glucose, D-mannose, D-glucuronate in the molar ratio of 2.8:2.0:20, and is partially acetylated with about 4.7% acetyl. Xanthan gum also includes about 3% pyruvate, which is attached to a single unit D-glucopyromosyl side chain as a metal. It dissolves in hot or cold water and the viscosity of aqueous solutions of xanthan gum is only slightly affected by changes in the pH of a solution between 1 and 11.

The term "heteropolysaccharide" as used in the present invention is defined as a water-soluble polysaccharide containing two or more kinds of sugar units, the heteropolysaccharide having a branched or helical configuration, and having excellent water-wicking properties and immense thickening properties. When admixed with an appropriate cationic cross-linking agent capable of cross-linking with the heteropolysaccharide in accordance with the present, invention and exposed to an aqueous solution, gastric fluid, etc., the gum packs closely and many intermolecular attachments are formed which make the structure strong and provide a hydrophilic gum matrix having high gel strength. The cationic cross-linking agent is therefore an agent capable of cross-linking the heteropolysaccharide, thus affecting the rate of release of the active medicament.

The cationic cross-linking agent may be monovalent or multivalent metal cations. The preferred salts are the inorganic salts, including various alkali metal and/or alkaline earth metal sulfates, chlorides, borates, bromides, citrates, acetates, lactates, etc. Specific examples of suitable cationic cross-linking agents include calcium sulfate, sodium chloride, potassium sulfate, sodium carbonate, lithium chloride, tripotassium phosphate, sodium borate, potassium bromide, potassium fluoride, sodium bicarbonate, calcium chloride, magnesium chloride, sodium citrate, sodium acetate, calcium lactate, magnesium sulfate and sodium fluoride or mixtures thereof. Multivalent metal cations may also be utilized. However, the preferred cationic cross-linking agents are bivalent. Particularly preferred salts are calcium sulfate and sodium chloride. The cationic cross-linking agents of the present invention are added in an amount effective to obtain a desirable increased gel strength due to the cross-linking.

Two steps which are generally required for gelation are the fast hydration of the macromolecules which comprise the hydrophilic material and thereafter the association of the molecules to form gels. Thus, two important properties of a hydrophilic gel matrix which are needed for application in a sustained-release system are the fast hydration of the system and a matrix having a high gel strength. As noted above, the cationic cross-linking agent may affect the hydration process of the heteropolysaccharide. These two important properties which are needed for application in a sustained-release system are the fast hydration of the system and a matrix having a high gel strength. These two important properties which are necessary to achieve a slow release hydrophilic matrix are maximized in the present invention by the particular combination of materials. In particular, heteropolysaccharides such as xanthan gum have excellent water wicking properties which provide fast hydration. On the other hand, the combination of xanthan gum with cationic cross-linking materials and the like which are capable of cross-linking the rigid helical ordered structure of the xanthan gum and can alter the gelation process and thus effect the rate of release of the active medicament.

In the present invention, it has been discovered that the controlled release properties of the tablets are optimized when the ratio of xanthan gum to cationic cross-linking agent (e.g., calcium sulfate, etc.) is from 1:1 to 3.5:1 and most preferably from 1.5:1 to 3:1, although xanthan gum in an amount of from 15 to 30 percent or more by weight of the sustained release excipient provides an acceptable slow release product.

In one preferred embodiment, the cationic cross-linking agent comprises calcium sulfate, and is present in the sustained release excipient in an amount of about 10 percent, by weight of the excipient. The ratio of the heteropolysaccharide to the cationic cross-linking agent is preferably from 1.5:1 to 3:1.

Any generally accepted soluble or insoluble inert pharmaceutical filler (diluent) material can be used. Preferably, the inert pharmaceutical filler comprises a monosaccharide, a disaccharide, a polyhydric alcohol, a cellulose (such as microcrystalline cellulose) and/or mixtures thereof. Examples of suitable inert pharmaceutical fillers include sucrose, dextrose, lactose, microcrystalline cellulose, xylitol, fructose, sorbitol, starches, mixtures thereof and the like. However, it is preferred that a soluble pharmaceutical filler such as dextrose, sucrose, or mixtures thereof be used.

In certain preferred embodiments of the invention, the sustained release matrix further comprises a hydrophobic material in an amount effective to slow the hydration of the gum without disrupting the hydrophilic matrix formed by the heteropolysaccharide when the formulation is exposed to fluids in an environment of use. This may be accomplished by granulating the sustained release matrix with a solution or dispersion of hydrophobic material prior to the incorporation of the medicament. The hydrophobic material may be selected from ethylcellulose, acrylic and/or methacrylic acid polymers or copolymers, hydrogenated vegetable oils, zein, as well as other pharmaceutically acceptable hydrophobic materials known to those skilled in the art. Other hydrophobic cellulosic materials such as other alkyl celluloses'may also be used. The amount of hydrophobic material incorporated into the sustained release matrix is that which is effective to slow the hydration of the gums without disrupting the hydrophilic matrix formed upon exposure to an environmental fluid. In certain preferred embodiments of the present invention, the hydrophobic material may be included in the sustained release matrix in an amount from 1% to 20% by weight. More preferably, the hydrophobic material may be included in the sustained release matrix in an amount from 3% to 12%, and most preferably from 5% to 10%, by weight of the final formulation. The hydrophobic material may be dissolved in an organic solvent or dispersed in an aqueous solution for incorporation into the formulation.

The combination of the hydrophilic material (xanthan gum) with the cationic cross-linking agent and inert diluent provides a ready to use sustained-release excipient in which a formulator need only blend the desired active medicament and an optional lubricant with the excipient and then make an oral solid dosage form. The sustained-release excipient may thus comprise a physical admix of the heteropolysaccharide along with a cationic cross-linking agent, or soluble excipient such as sucrose, lactose or dextrose.

One of the limitations of direct compression as a method of tablet manufacture is the size of the tablet. For example, where the dosage form is an oral sustained release tablet and the dose of therapeutically active agent to be contained in the tablet is relatively large, a pharmaceutical formulator may choose to wet granulate the drug with other excipients to attain a desired tablet size with the correct compact strength (e.g., hardness). Usually the amount of filler/binder or excipients needed in wet granulation is less than that in direct compression since the process of wet granulation contributes to some extent toward the desired physical properties of a tablet. Accordingly, the sustained release pharmaceutical excipient prepared in accordance with the present invention may be subjected to wet granulation before the medicament is added. In this technique, the desired amounts of the heteropolysaccharide, the cationic cross-linking agent, and the inert filler are mixed together and thereafter a moistening agent such as water, propylene glycol, glycerol, alcohol or the like is added to prepare a moistened mass. Next, the moistened mass is dried. The dried mass is then milled with conventional equipment into granules. Therefore, the sustained-release excipient product is ready to use. The granulate thus obtained has certain advantages including the fact that it is free-flowing, has good or hesive properties, and can be admixed with an active agent (e.g., drug) and can be directly compressed into tablets. On the other hand, the granulate can be formulated into a capsule, used in the granulate form, extruded, and/or spheronized with an active medicament to form pellets, etc.

Alternatively, it is possible to dry mix the ingredients of the sustained release excipient without utilizing a wet granulation step. This procedure may be utilized, for example, where a wet granulation step is to be accomplished when the active ingredient is directly added to the ingredients of the sustained release excipient. On the other hand, this procedure may also be used where no wet granulation step whatsoever is contemplated. If the mixture is to be manufactured without a wet granulation step, and the final mixture is to be tabletted, it is preferred that all or part of the inert diluent comprise a pre-manufactured direct compression diluent. Such direct compression diluents are widely used in the pharmaceutical arts, and may be obtained from a wide variety of commercial sources. Examples of such pre-manufactured direct compression excipients include Emcocel® (microcrystalline cellulose, N.F.), Emdex® (dextrates, N.F.), and Tab-Fine® (a number of direct-compression sugars including sucrose, fructose, and dextrose), all of which are commercially available from Edward Mendell Co., Inc., Patterson, New York). Other direct compression diluents include Anhydrous lactose (Lactose N.F., anhydrous direct tabletting) from Sheffield Chemical, Union, N.J. 07083; Elcems® G-250 (Powdered cellulose, N.F.) from Degussa, D-600 Frankfurt (Main) Germany; Fast-Flo Lactose® (Lactose, N.F., spray dried) from Foremost Whey Products, Banaboo, WI 53913; Maltrin® (Agglomerated maltrodextrin) from Grain Processing Corp., Muscatine, IA 52761; Neosorb 60® (Sorbitol, N.F., direct-compression) from Roquette Corp., 645 5th Ave., New York, NY 10022; Nu-Tab® (Compressible sugar, N.F.) from Ingredient Technology, Inc., Pennsauken, NJ 08110; Polyplasdone XL® (Crospovidone, N.F., cross-linked polyvinylpyrrolidone) from GAF Corp., New York, NY 10020; Primojel® (Sodium starch glycolate, N.F., carboxymethyl starch) from Generichem Corp., Little Falls, NJ 07424; Solka Floc® (Cellulose floc) from Edward Mendell Co., Carmel, NY 10512; Spray-dried lactose® (Lactose N.F., spray dried) from Foremost Whey Products, Baraboo, WI 53913 and DMV Corp., Vehgel, Holland; and Sta-Rx 1500® (Starch 1500) (Pregelatinized starch, N.F., compressible) from Colorcon, Inc., West Point, PA 19486.

In general, the formulator may prepare a directly compressible diluent, by wet granulating or spray drying lactose, for example. For purposes of the present invention, these specially treated inert diluents will be referred to as "directly compressible" inert diluents.

In further embodiments of the present invention, the directly compressible inert diluent which is used in conjunction with the sustained release pharmaceutical excipient of the present invention is an augmented microcrystalline cellulose as disclosed in U.S. Patent Application Serial No. 08/370,576, filed January 9, 1995, and entitled "PHARMACEUTICAL EXCIPIENT HAVING IMPROVED COMPRESSIBLITY", inventors J. Staniforth, B. Sherwood and E. Hunter.

Once the sustained release excipient of the present invention has been prepared, it is then possible to blend the same with an active medicament, metoprolol, e.g., in a V-blender. The mixture may then be manufactured into the desired final dosage form. If desired, the mixture can be directly compressed into tablets, or subjected to other intermediate processing steps such as wet granulation.

The dosage forms of the present invention are preferably tablets. However, the ingredients may also be formulated in a capsule, extruded and spheronized with an active medicament to form pellets, etc.

For example, the complete mixture, in an amount sufficient to make a uniform batch of tablets, is then subjected to tabletting in a conventional production scale tabletting machine at normal compression pressure, i.e. about 1.379 x 10⁷ to 1.103 x 10⁷ Pa (2000-1600 lbs/sq in). However, the mixture should not be compressed to such a degree that there is subsequent difficulty in its hydration when exposed to gastric fluid. An effective amount of any generally accepted pharmaceutical lubricant, including the calcium or magnesium soaps may be added to the above-mentioned ingredients of the excipient be added at the time the medicament is added, or in any event prior to compression into a said dosage form. One preferred lubricant is Pruv® , e.g., in the amount of about 3.0 percent of the solid dosage form.

The average tablet size for round tablets is preferably 500 mg to 750 mg and for capsule-shaped tablets 750 mg to 1000 mg.

The average particle size of the granulated excipient of the present invention ranges from 50 microns to 400 microns and preferably from 185 microns to 265 microns. The particle size of the granulation is not narrowly critical, the important parameter being that the average particle size of the granules, must permit the formation of a directly compressible excipient which forms pharmaceutically acceptable tablets. The desired tap and bulk densities of the granulation of the present invention are normally between from 0.3 to 0.8 g/ml, with an average density of from 0.5 to 0.7 g/ml. For best results, the tablets formed from the granulations of the present invention are from 6 to 8 kg hardness. The average flow of the granulations prepared in accordance with the present invention are from 25 to 40 g/sec.

Variables which may affect the release rate and the compressibility of tablets prepared with the excipient of the present invention are the drug to gum ratio; the method of incorporation of excipient (method of granulation); the relative amount of the gum to cationic cross-linking agent; and the ratio of active medicament to the sustained-release excipient.

The sustained release excipient formulations of the present invention may be utilized in the preparation of a wide range of 24 hour solid dosage forms which include a wide range of water-soluble or water-insoluble medicaments. Examples of such therapeutically active agents include antihistamines (e.g., dimenhydrinate, diphenhydramine, chlorpheniramine and dexchlorpheniramine maleate), analgesics (e.g., aspirin, codeine, morphine, dihydromorphone, oxycodone, etc.), anti-inflammatory agents (e.g., naproxen, diclofenac, indomethacin, ibuprofen, aspirin, sulindac), acetaminophen, gastro-intestinals and anti-emetics (e.g., metoclopramide), anti-epileptics (e.g., phenytoin, meprobamate and nitrezepam), vasodilators (e.g., nifedipine, papaverine, diltiazem and nicardipine), anti-tussive agents and expectorants (e.g., codeine phosphate), anti-asthmatics (e.g. theophylline), anti-spasmodics (e.g., atropine, scopolamine), hormones (e.g., insulin, heparin), diuretics (e.g., ethacrynic acid, bendroflumethiazide), anti-hypertensives (e.g., propranolol, clonidine), bronchodilators (e.g., albuterol), anti-inflammatory steroids (e.g., hydrocortisone, triamcinolone, prednisone), antibiotics (e.g., tetracycline), antihemorrhoidals, hypnotics, psychotropics, antidiarrheals, mucolytics, sedatives, decongestants, laxatives, antacids, vitamins, stimulants (including appetite suppressants such as phenylpropanolamine).

Upon oral ingestion and contact with gastric fluid, the controlled release tablets prepared according to the present invention swell and gel to form a hydrophilic gel matrix from which the drug is released. The swelling of the matrix causes a reduction in the bulk density of the tablet and provides the buoyancy necessary to allow the gel mass to float on the stomach contents to provide a slow delivery of the medicament. The matrix, the size of which is dependent upon the size of the original tablet, can swell considerably and become obstructed near the opening to the pylorus. Since the medicament is dispersed throughout the tablet (and consequently throughout the gel matrix), a constant amount of drug can be released per unit time in vivo by dispersion or erosion of the outer portions of the matrix. This phenomenon is commonly referred to as a zero order release profile or zero order kinetics. The process continues, with the matrix remaining buoyant in the stomach, until substantially all of the medicament is released. The chemistry of certain of the ingredients comprising the excipients of the present invention such as xanthan gum is such that the excipients are considered to be self-buffering agents which are substantially insensitive to the solubility of the medicament and likewise insensitive to the pH changes along the length of the gastrointestinal tract. Moreover, the chemistry of the ingredients comprising the excipients of the present invention is believed to be similar to certain known mucoadhesive substances such as polycarbophil. Mucoadhesive properties are desirable for buccal delivery systems. Thus, it may be possible that the gel system could potentially loosely interact with the mucin in the gastrointestinal tract and thereby provide another mode by which a constant rate of delivery of the medicament is achieved. The above hypothesis is included for discussion purposes only.

These two phenomenons, i.e., buoyancy of the gel matrix and the mucoadhesive properties discussed above, are possible mechanisms by which the gel matrix of the present invention could interact with the mucin and fluids of the gastrointestinal tract and provide a constant rate of delivery of the medicament.

### DETAILED DESCRIPTION OF THE PREFERRED EMBODIMENTS

The following examples illustrate various aspects of the present invention.

### EXAMPLES 1-3

The sustained release excipient is prepared by dry blending the requisite amounts of xanthan gum, dextrose and calcium sulfate in a high-speed mixer/granulator for 2 minutes. While running choppers/impellers, the water is added and the mixture is granulated for another 2 minutes. The granulation is then dried in a fluid bed dryer to a loss on drying weight (LOD) of between 4 and 7%. The granulation is then milled using 20 mesh screens. The ingredients of the sustained release excipient of Example 1 are set forth in Table 1 below:

**TABLE 1**

| **PREPARATION OF SUSTAINED RELEASE EXCIPIENT** | | | |
|---|---|---|---|
| Component | %-Ex. 1 | %-Ex. 2 | %-Ex. 3 |
| 1. Xanthan gum | 30 | 15 | 30 |
| 2. Dextrose | 60 | 75 | 70 |
| 3. Calcium Sulfate | 10 | 10 | 0 |
| 4. Water | 10* | 10* | 10* |

| | | | |
|---|---|---|---|
| *removed during processing | | | |

Next, the sustained release excipient prepared as detailed above is dry blended with a desired amount of medicament (in the following examples metoprolol, provided as the tartrate salt) in a V-blender for 10 minutes. A suitable amount of tabletting lubricant Pruv® (sodium stearyl fumarate, NF, commercially available from the Edward Mendell Co., Inc.) for the following examples is added and the mixture is blended for another 5 minutes. This final mixture is compressed into tablets, each tablet containing 100 mg metoprolul. The tablets of Example 1 weighed 618.5 mg. The tablets of Example 2 weighed 618.5 mg. The tablets of Example 3 weighed 618.5 mg. The drug:gum ratio of the tablets of Example 1 was 1:1.5. The drug:gum ratio of the tablets of Example 2 was 1:0.75. The drug:gum ratio of the tablets of Example 3 was 1:1.5. The ingredients of the tablets of Examples 1-3 are set forth in Table 2 below:

**TABLE 2**

| Component | % |
|---|---|
| 1. Sustained Release Excipient | 80.8% |
| 2. Metoprolol | 16.2% |
| 3. Pruv® | 3.0% |

Dissolution tests were then carried out on the tablets of Examples 1-3. The dissolution tests are conducted in an automated USP dissolution apparatus (Paddle Type II, pH 6.8 buffer, 100 rpm.). The results are set forth in Table 3 below:

**TABLE 3**

| **Effect of Single Gum Composition** | | | |
|---|---|---|---|
| Time(hours) | Example 1 | Example 2 | Example 3 |
| 0 | 0.0 | 0.0 | 0.0 |
| 2 | 25.3 | 29.0 | 20.7 |
| 4 | 37.9 | 42.7 | 32.3 |
| 8 | 56.3 | 63.6 | 50.2 |
| 12 | 70.6 | 77.9 | 64.1 |
| 16 | 81.3 | 88.2 | 74.3 |
| 20 | 89.0 | 94.9 | 81.3 |
| 24 | 97.6 | 98.8 | - |

From the results provided in Table 3, it can be seen that formulations made with a greater concentration of gum produced slower drug release rates. It is also evident that the incorporation of calcium sulfate into single gum systems results in a faster drug release rates compared to formulations without calcium sulfate. Accordingly, the results provide that the tablets in Example 1 are suitable for delivering medicaments as an oral solid dosage form over a 24-hour oral period of time.

For the avoidance of doubt, it is confirmed that the formulation of Example 3 does not form part of the claimed invention.

## Claims

1. A sustained release oral solid dosage form, comprising a therapeutically active agent and a sustained release excipient, the excipient comprising:
a gum component consisting of xanthan gum, the percentage of said gum being from 10 to 40 percent of the sustained release excipient;
a cationic cross-linking agent capable of cross-linking said gum in the presence of aqueous solutions, the ratio of said gum to said cationic cross-linking agent being from 1:1 to 3.5:1; and
an inert pharmaceutical diluent selected from the group consisting of a monosaccharide, a disaccharide, a polyhydric alcohol, a cellulose, a starch, and mixtures thereof, the percentage of said inert diluent being from 60 to 85 percent by weight, of the sustained release excipient.

2. An oral dosage form of claim 1, wherein said inert pharmaceutical diluent is selected from the group consisting of lactose, dextrose, sucrose, fructose, microcrystalline cellulose, xylitol, sorbitol and mixtures thereof.

3. An oral dosage form of claim 1, wherein the ratio of said inert pharmaceutical diluent to said gum is from 6:1 to 2:1.

4. An oral dosage form of claim 1, wherein the cationic cross-linking agent comprises from 1 to 20 percent of said sustained release excipient, by weight.

5. An oral dosage form of claim 1, wherein said cationic cross-linking agent is selected from the group consisting of calcium sulfate, sodium chloride, potassium sulfate, sodium carbonate, lithium chloride, tripotassium phosphate, sodium borate, potassium bromide, potassium fluoride, sodium bicarbonate, calcium chloride, magnesium chloride, sodium citrate, sodium acetate, calcium lactate, magnesium sulfate, sodium fluoride and mixtures thereof.

6. An oral dosage form of claim 1, wherein said cationic cross-linking agent is calcium sulfate.

7. An oral dosage form of claim 1, wherein said excipient is in the form of a granulate.

8. An oral dosage form of claim 1, wherein said inert pharmaceutical diluent is in a directly compressible form.

9. An oral dosage form of claim 1, wherein said gum, said inert pharmaceutical diluent, and said cationic cross-linking agent are granulated with a hydrophobic material selected from the group consisting of an alkylcellulose, a copolymer of acrylic and methacrylic acid esters, waxes, shellac, zein, hydrogenated vegetable oils, and mixtures of any of the foregoing, said hydrophobic material being included in an amount effective to slow the hydration of said gelling agent when exposed to an environmental fluid.

10. An oral dosage form of claim 9, wherein said hydrophobic material is present in an amount from 1 to 20 percent, by weight of said sustained release excipient.

11. An oral dosage form of claim 9, wherein said hydrophobic material is ethylcellulose.

12. An oral dosage form of any one of claims 1 to 11, wherein the sustained release excipient comprises the gum, cationic cross-linking agent, and the inert pharmaceutical diluent having been agglomerated into granular particles via a wet granulation method.

13. An oral dosage form of claim 1, wherein said dosage form is a tablet.

14. An oral dosage form of claim 1, wherein said dosage form is a capsule.

15. A method for preparing a sustained release oral solid dosage form of any one of claims 1-14, comprising:
preparing the sustained release excipient by mixing the gum, the cationic cross-linking agent and the inert pharmaceutical diluent;
subsequently adding the active agent; and
forming the mixture into a solid oral dosage form.

16. A method of claim 15, wherein the method further comprises:
wet granulating the gum, the cationic cross-linking agent and the inert pharmaceutical diluent;
drying the mixture to obtain a granulate of distinct excipient particles;
mixing the active agent with the granulate; and
forming the mixture into a solid dosage form.

17. A method of claim 15, wherein said cationic cross-linking agent is calcium sulfate.

18. A method of claim 15, wherein the gum, said inert pharmaceutical diluent, and said cationic cross-linking agent are granulated with a hydrophobic material selected from the group consisting of an alkylcellulose, a copolymer of acrylic and methacrylic acid esters, waxes, shellac, zein, hydrogenated vegetable oils, and mixtures of any of the foregoing, said hydrophobic material being included in an amount effective to slow the hydration of said gelling agent when exposed to an environmental fluid.

## Patentansprüche

1. Feste orale Dosierform mit anhaltender Abgabe, umfassend ein therapeutisch wirksames Mittel und ein Retard-Excipiens, wobei das Excipiens umfasst:
einen Gummibestandteil bestehend aus Xanthangummi, wobei der Prozentanteil des Gummis 10 bis 40 Prozent des Retard-Excipiens beträgt;
ein kationisches quervernetzendes Mittel, das in der Lage ist, das Gummi in Gegenwart von wässrigen Lösungen querzuvernetzen, wobei das Verhältnis des Gummis zu dem kationischen quervernetzenden Mittel 1:1 bis 3,5:1 beträgt; und
ein inertes pharmazeutisches Diluens, ausgewählt aus der Gruppe, bestehend aus einem Monosaccharid, einem Disaccharid, einem Polyhydroxyalkohol, einer Zellulose, einer Stärke und Mischungen davon, wobei der Prozentanteil des inerten Diluens 60-85 Gewichtsprozent des Retard-Excipiens beträgt.

2. Orale Dosierform nach Anspruch 1, wobei das inerte pharmazeutische Diluens ausgewählt ist aus der Gruppe, bestehend aus Laktose, Dextrose, Saccharose, Fruktose, mikrokristalliner Zellulose, Xylit, Sorbit und Mischungen davon.

3. Orale Dosierform nach Anspruch 1, wobei Verhältnis des inerten pharmazeutischen Diluens zu dem Gummi 6:1 bis 2:1 beträgt.

4. Orale Dosierform nach Anspruch 1, wobei das kationische quervernetzende Mittel 1 bis 20 Gewichtsprozent des Retard-Excipiens umfasst.

5. Orale Dosierform nach Anspruch 1, wobei das kationische quervernetzende Mittel ausgewählt ist aus der Gruppe, bestehend aus Kalziumsulfat, Natriumchlorid, Kaliumsulfat, Natriumkarbonat, Lithiumchlorid, Trikaliumphosphat, Natriumborat, Kaliumbromid, Kaliumfluorid, Natriumbikarbonat, Kalziumchlorid, Magnesiumchlorid, Natriumcitrat, Natriumactetat, Kalziumlaktat, Magnesiumsulfat, Natriumfluorid und Mischungen davon.

6. Orale Dosierform nach Anspruch 1, wobei das kationische quervernetzende Mittel Kalziumsulfat ist.

7. Orale Dosierform nach Anspruch 1, wobei das Excipiens in Form eines Granulats vorliegt.

8. Orale Dosierform nach Anspruch 1, wobei das inerte pharmazeutische Diluens in einer direkt verpressbaren Form vorliegt.

9. Orale Dosierform nach Anspruch 1, wobei das Gummi, das inerte pharmazeutische Diluens und das kationische quervernetzende Mittel granuliert sind mit einem hydrophoben Material, ausgewählt aus der Gruppe, bestehend aus einer Alkylzellulose, einem Copolymer aus Acryl- und Methacrylsäureestern, Waxen, Schellack, Zein, hydrierten Pflanzenölen und Mischungen von jedem vorstehend genannten, wobei das hydrophobe Material in einer Menge enthalten ist, die wirksam ist, um die Hydratation des Geliermittels bei Exposition gegenüber einer Umgebungsflüssigkeit zu verlangsamen.

10. Orale Dosierform nach Anspruch 9, wobei das hydrophobe Material in einer Menge von 1-20 Gewichtsprozent des Retard-Excipiens zugegen ist.

11. Orale Dosierform nach Anspruch 9, wobei das hydrophobe Material Ethylzellulose ist.

12. Orale Dosierform nach einem der Ansprüche 1 bis 11, wobei das Retard-Excipiens das Gummi, kationische quervernetzende Mittel und das inerte pharmazeutische Diluens umfasst, die mittels eines Feuchtgranulierungsverfahrens in granuläre Partikel granuliert worden sind.

13. Orale Dosierform nach Anspruch 1, wobei die Dosierform eine Tablette ist.

14. Orale Dosierform nach Anspruch 1, wobei die Dosierform eine Kapsel ist.

15. Verfahren zur Herstellung einer festen oralen Dosierform mit anhaltender Abgabe nach einem der Ansprüche 1-14, umfassend
das Herstellen des Retard-Excipiens durch Mischen des Gummis, des kationischen quervernetzenden Mittels und des inerten pharmazeutischen Diluens;
die anschließende Zugabe des wirksamen Mittels; und das Formen der Mischung zu einer festen oralen Dosierform.

16. Verfahren nach Anspruch 15, wobei das Verfahren ferner umfasst:
das Feuchtgranulieren des Gummis, des kationischen quervernetzenden Mittels und des inerten pharmazeutischen Diluens;
das Trocknen der Mischung, um ein Granulat von getrennten Excipiens-Partikeln zu erhalten;
das Mischen des wirksamen Mittels mit dem Granulat; und
das Formen der Mischung zu einer festen Dosierform.

17. Verfahren nach Anspruch 15, wobei das kationische quervernetzende Mittel Kalziumsulfat ist.

18. Verfahren nach Anspruch 15, wobei das Gummi, das inerte pharmazeutische Diluens und das kationische quervernetzende Mittel granuliert werden mit einem hydrophoben Material, ausgewählt aus der Gruppe, bestehend aus einer Alkylzellulose, einem Copolymer aus Acryl- und Methacrylsäureestern, Waxen, Schellack, Zein, hydrierten Pflanzenölen und Mischungen von jedem vorstehend genannten, wobei das hydrophobe Material in einer Menge enthalten ist, die wirksam ist, um die Hydratation des Geliermittels bei Exposition gegenüber einer Umgebungsflüssigkeit zu verlangsamen.

## Revendications

1. Forme posologique solide orale à libération prolongée, comprenant un agent thérapeutiquement actif et un excipient à libération prolongée, l'excipient comprenant :
un constituant du type gomme, consistant en gomme xanthane, le pourcentage de ladite gomme représentant 10 à 40 % de l'excipient à libération prolongée ;
un agent de réticulation cationique apte à la réticulation de ladite gomme en présence de solutions aqueuses, le rapport de ladite gomme audit agent de réticulation cationique étant compris dans l'intervalle de 1:1 à 3,5:1 ; et
un diluent pharmaceutique inerte choisi dans le groupe consistant en un monosaccharide, un disaccharide, un alcool polyhydroxylique, une cellulose, un amidon et leurs mélanges, le pourcentage dudit diluent inerte étant compris dans l'intervalle de 60 à 85 % en poids de l'excipient à libération prolongée.

2. Forme posologique orale suivant la revendication 1, dans laquelle ledit diluant pharmaceutique inerte est choisi dans le groupe consistant en lactose, dextrose, saccharose, fructose, cellulose microcristalline, xylitol, sorbitol et leurs mélanges.

3. Forme posologique orale suivant la revendication 1, dans laquelle le rapport dudit diluent pharmaceutiquement inerte à ladite gomme est compris dans l'intervalle de 6:1 à 2:1.

4. Forme posologique orale suivant la revendication 1, dans laquelle l'agent de réticulation cationique représente 1 à 20 % dudit excipient à libération prolongée, en poids.

5. Forme posologique orale suivant la revendication 1, dans laquelle ledit agent de réticulation cationique est choisi dans le groupe consistant en sulfate de calcium, chlorure de sodium, sulfate de potassium, carbonate de sodium, chlorure de lithium, phosphate tripotassique, borate de sodium, bromure de potassium, fluorure de potassium, bicarbonate de sodium, chlorure de calcium, chlorure de magnésium, citrate de sodium, acétate de sodium, lactate de calcium, sulfate de magnésium, fluorure de sodium et leurs mélanges.

6. Forme posologique orale suivant la revendication 1, dans laquelle ledit agent de réticulation cationique est le sulfate de calcium.

7. Forme posologique orale suivant la revendication 1, dans laquelle ledit excipient est sous forme de granules.

8. Forme posologique orale suivant la revendication 1, dans laquelle ledit diluent pharmaceutique inerte est sous une forme directement compressible.

9. Forme posologique orale suivant la revendication 1, dans laquelle ladite gomme, ledit diluent pharmaceutique inerte et ledit agent de réticulation cationique sont granulés avec une matière hydrophobe choisie dans le groupe consistant en une alkylcellulose, un copolymère d'esters d'acide acrylique et d'acide méthacrylique, des cires, la gomme laque, la zéine, des huiles végétales hydrogénées et des mélanges de n'importe lesquelles des matières précitées, ladite matière hydrophobe étant incluse dans une quantité efficace pour ralentir l'hydratation dudit agent gélifiant lors de l'exposition à un fluide ambiant.

10. Forme posologique orale suivant la revendication 9, dans laquelle ladite matière hydrophobe est présente en une quantité de 1 à 20 %, en poids dudit excipient à libération prolongée.

11. Forme posologique orale suivant la revendication 9, dans laquelle ladite matière hydrophobe est l'éthylcellulose.

12. Forme posologique orale suivant l'une quelconque des revendications 1 à 11, dans laquelle l'excipient à libération prolongée comprend la gomme, l'agent de réticulation cationique et le diluent pharmaceutique inerte ayant été agglomérés en particules granulaires par un procédé de granulation par voie humide.

13. Forme posologique orale suivant la revendication 1, ladite forme posologique étant un comprimé.

14. Forme posologique orale suivant la revendication 1, ladite forme posologique étant une capsule.

15. Procédé pour la préparation d'une forme posologique solide orale à libération prolongée de l'une quelconque des revendications 1 à 14, comprenant les étapes consistant :
à préparer l'excipient à libération prolongée en mélangeant la gomme, l'agent de réticulation cationique et le diluent pharmaceutique inerte ;
puis à ajouter l'ingrédient actif ; et
à transformer le mélange en une forme posologique orale solide.

16. Procédé suivant la revendication 15, ledit procédé comprenant en outre les étapes consistant :
à granuler par voie humide la gomme, l'agent de réticulation cationique et le diluent pharmaceutique inerte ;
à sécher le mélange pour obtenir des granules de particules d'excipient distinctes ;
à mélanger l'agent actif aux granules ; et
à transformer le mélange en une forme posologique solide.

17. Procédé suivant la revendication 15, dans lequel ledit agent de réticulation cationique est le sulfate de calcium.

18. Procédé suivant la revendication 15, dans lequel la gomme, ledit diluent pharmaceutique inerte et ledit agent de réticulation cationique sont granulés avec une matière hydrophobe choisie dans le groupe consistant en une alkylcellulose, un copolymère d'esters d'acide acrylique et d'acide méthacrylique, des cires, la gomme laque, la zéine, des huiles végétales hydrogénées et des mélanges de n'importe lesquelles des matières précitées, ladite matière hydrophobe étant incluse en une quantité efficace pour ralentir l'hydratation dudit agent gélifiant lors de l'exposition à un fluide ambiant.
